(19)

Europäisches Patentamt
European Patent Office
Office européen des brevets

(11) **EP 2 735 607 A1**

(12) **EUROPEAN PATENT APPLICATION**

(43) Date of publication:
28.05.2014 Bulletin 2014/22

(21) Application number: 12464023.6

(22) Date of filing: 29.11.2012

(51) Int Cl.:
*C12N 1/14* (2006.01)     *A01N 63/00* (2006.01)
*C12R 1/885* (2006.01)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(30) Priority: 27.11.2012 RO 201200890

(83) Declaration under Rule 32(1) EPC (expert solution)

(71) Applicant: SC Corx Bioneer Ceu SA
530104 Miercurea-Ciuc, Jud. Harghita (RO)

(72) Inventors:
• Oancea, Florin
  062082 Bucharest (RO)

• Mara, Gyöngyvér
  530100 Miercurea Ciuc
  Jud. Harghita (RO)
• Sesan, Tatiana Eugenia
  061079 Bucharest (RO)
• Máthé, István
  530100 Miercurea Ciuc
  Jud. Harghita (RO)
• Raut, Juliana
  032768 Bucharest (RO)
• Ábrahám, Beáta
  530103 Miercure Ciuc
  Jud. Harghita (RO)
• Lányi, Szabolcs
  530174 Miercurea Ciuc
  Jud. Harghita (RO)

(54) **Strain of Trichoderma harzianum and controlled release composition which contains said strain**

(57)     The invention relates to a strain of *Trichoderma harzianum* Td50b, deposited with the deposit number NCAIM (P) F 001412, which produces the exohydrolases involved in the degradation of plant material and presents antagonism against pathogens of ornamental crops, including through synthesis of odorant volatile compounds with antifungal activity. The strain was used to produce a controlled release compositions that promote the colonization of the material intended to be composted, assuring the survival of propagules not-liberated after the initial colonization of the substrate with the strain producing fungistatic volatiles compounds, including for the dormant propagules of the same strain, and, on the end, accelerate the obtainment process of composts intended to be used for mulching of the ornamental crop soil, in order to protect them against the attack of pathogen agents.

EP 2 735 607 A1

**Description**

The present invention refer to a strain of *Trichoderma harzianum* which protect the ornamentals plants for some diseases, produce exohydrolases with a role in degradation of the vegetable material and synthetize odorant volatile compounds, and to a controlled release composition which contain said strain, intended to accelerate the production of suppressive and odorant composts, used as mulch for the protection and nutrition of ornamentals crops.

There are known a number of strains of microscopic fungi belonging to the *Trichoderma* genera, which are antagonist for the phytopathogen agents and which have a high saprophytic competence, colonizing various substrates and conferring to these suppressive characteristics for plant diseases. Patent EP1400586 (B1) describe a strain of *Trichoderma asperellum,* T34 (2), with the deposit number CECT 20147, Coleccion Espaniola de Cultivos Tipo, Valencia, Spain, which have the capacity to colonize growing substrate for plant, for example peat, compost (composted hardwood bark, composted pine bark, composted cork, composted sewage sludge, composted yard wastes, etc.) or formulation of CPV (compost - peat - vermiculite). The substrate resulted by colonization with this strain present the advantage to be suppressive to both *Fusarium oxysporum* f.sp. *lycopersici* and *Rhizoctonia solanii.* The claimed process for production of the suppressive substrate involve the distribution of an effective amount, from the strain T34 (2), in the growing substrate, to suppress the attack of the phytopathogen agents. Patent US 7070984 (B2) refer to the strain Li49 of *T. viride,* deposited to ATCC under the number PTA-1225, which have an increased capacity to colonize different substrates, and which acts like a parasite of the phytopathogen agents, especially of those from *Fusarium* and *Rhizocotonia* genera. The strain is aseptically cultivated on a liquid medium and the biomass is recovered and added in proportion of at least 10% in an organic support made from cereal grains, peat and compost. The resulted bio-preparation is used for treatment against the soil phytopathogen, in a dose of 5 pounds per 1 acre (5.6 kg/ha). Patent US 4900348 claims the use of strains of *Trichoderma hamatum* ATCC 20764 or 20765, in combination with bacteria *Xanthomonas maltophila* ATCC 53199 and *Flavobacterium balustinum* ATCC 53199, for the production of a suppressive compost for *Rhizoctonia solanii, Pythium ultimum* and *Fusarium,* starting from hardwood bark, pine bark, sewage sludge as a composting substrate.

The strains of *Trichoderma* have also a significant activity for degradation of vegetable material, due to production of hydrolytic enzymes, especially cellulases. Patent TWI287535 (B) present the strain TC103 of *Trichoderma* spp., deposited under number BCRC 930070 on Bioresources Collection and Research Center, Taipei, which have the capacity to accelerate the degradation of bagasse / wood residues with compost formation. Patent Application RO 127471 (A1) protect the strain Td85 of *T. pseudokoningii,* deposit number DSM 23661, which concomitantly present antagonist activity against soil phytopathogen agents, high capacity for mineralization of the vegetable material and resistance to the biofumigant compounds released from the cruciferous biomass, and which is intended to accelerate the mineralization of the vegetable biomass resulted from the termination of the cruciferous "green" cover crops, used for soil protection during winter.

For some of the *Trichoderma* strains were revealed the capacity to produce odorant compounds, for example 6-pentyl-pyrone, which have also a significant antifungal activity. Patent Application WO9520879 (A2) claim the use of antifungal metabolites produced by different strains of *Trichoderma,* 6-pentyl-alpha-pyrone, delta-decalactone and massoio-lactone, alone or in combination with the strain which produce such metabolites, for controlling of the fungal diseases of cultivated plants.

There were not yet revealed strain of *Trichoderma,* active for the protection of ornamentals crops against some diseases, and which present in the same time: (*i*) capacity to produce cellulases / ß-glucanases, with function on acceleration of the decay of vegetable material from the composting substrate, and (*ii*) synthesis of volatiles compounds which are, in the same times, antifungal and odorants. The antagonist strains from this category, which have the capacity to synthetize exohydrolases involved in the degradation of the vegetable material and antifungal odorant compounds, could allow the formation of suppressive and odorant composts, useful as mulch for protection and nutrition of the ornamentals plant cultivates on the green urban spaces.

For such strains it is necessary also to develop composition with controlled release, which should promote the colonization of the material for compostation, and especially the tree bark which is used as organic mulch for gardens and green spaces. Pentyl-pyrones produced by the strains of *Trichoderma* have a fungistatic effect also on the vegetative cells / propagules from the same strain (Sahay-Bagnon et al., 2000, Process Biochem., 36:103-109), further colonization by the same strain of a substrate where is already developing a population producing 6-pentyl-pyrone being limited.

This invention refer to a strain of *Trichoderma harzianum* Td50b, deposited with the deposit number NCAIM (P) F 001412 at National Collection of Agricultural and Industrial Microorganisms, Corvinus University from Budapest, Hungary, which produce exohydrolases with role in degradation of the vegetable material and which present antagonism for some of the phytopathogen agents of the ornamentals crops, due inclusively to the synthesis of odorant compounds with antifungal activity. This invention also refer to a controlled release composition which: promote the colonization of the material intended to be composted, assuring the survival of propagules not-liberated after the initial colonization of the substrate with the strain producing fungistatic volatiles compounds, also for the dormant propagules of the same strain, and, on the end, accelerate the obtainment process of composts intended to be used for mulching of ornamental crop soil.

The strain of *Trichoderma harzianum* Td50b is antagonist *in vitro* against *Rhizoctonia solanii, Fusarium graminearum, F. culmorum, Pythium ultimum, Botrytis cinerea, Alternaria alternata, Sclerotinia sclerotiorum,* protect the ornamental crops for the damping-off diseases of seedlings produced by soil phytopathogen fungi (*Rhizoctonia, Pythium, Fusarium*) and for the rots producing fungi (*Botrytis cinerea*), produce cellulases with a role in the degradation of the vegetable material from the composting substrate, synthetize 6-pentyl-pryone, volatile compound which is, in the same time, anti-fungal and odorant, and accelerate the formation of the suppressive compost from pine bark.

The strain *T. harzianum* Td50b, deposit number NCAIM (P) F 001412, was selected from natural isolates by the use of a process for high throughput screening which include the following steps:

- Aseptically distribution of an agarized medium, which contain as principal source of carbon a cellulose derivative, in a 24-wells plate;
- Inoculation of an agarized medium with 5 isolated for testing, random distributed, maintaining several control not-inoculated wells;
- Coverage with a sterile plastic film which allow the gas exchange;
- Incubation for three days at 25°C;
- Deposition on the 24 well plates, on which were developed the tested isolates, over another plate with 24 wells with an agarized media, on which was grown for 2 days a microscopic fungus, phytopathogen agent, toward is tested the antagonism of the isolates;
- Incubation for 5 days at 25°C of the reunited plates, with the plates with isolates of *Trichoderma* over the plates with phytopathogenic fungus for testing, separated by a plastic film which allow the gas exchange;
- Determination of the phytopathogen agent growth by image analysis of the well plates, taken by a photodocumentation system, and calculation of the inhibition percent with the formula: $(A_1 - A_n)/A_1 \times 100$, where $A_1$ is the surface covered by the phytopathogen agent on the untreated control, and $A_n$ is the surface covered by the phytopathogen agent in that one of the experimental treatments wherein was confronted with the volatiles compounds produced by one of the tested isolates;
- Statistical analysis of the variance and identification of the eventual isolates active due to the production of volatiles compounds;
- Flooding of the agarized media on which were grown the isolates of *Trichoderma* with a solution of Red Congo 0.1 % and, after 5 min, removal of the solution of Red Congo and washing with a solution of 1 M NaCl for 20 min;
- Revealing of the clear zones of cellulolysis around the colonies of *Trichoderma* producing cellulases.

The controlled released composition based on the *T. harzianum* Td50b strain consists of 70 parts of hydrolyzed sawdust, 10 parts of flour, 4.8 parts of ethyl esters of fatty acids, 4 parts of polyvinyl alcohol, and 3.5 parts lecithin, 0.75 parts of potassium soap, 0.45 parts of glycerol, 0.4 parts triglycerides, residual water and min. $10^6$ cfu Td50b.

By the application of the invention are obtained the following advantages:

- Assurance of an uniform and reproducible colonization by the strain Td50b with propagules which are released sequentially from the composition according to present invention, due to preponderant accumulation in the hydrophobic component of the composition of 6-penty-pyrone, volatile compound, which is fungistatic for the propagules of the same strain and which are produced by the population already developed in the substrate from the first released waves;
- Acceleration of the process of obtainment suppressive and odorants composts by using the composition which release successively inoculant propagules of Td50b strain, producing high level of cellulases;
- Formation of suppressive compost for diseases of ornamentals cultivated plants and with superior odorant characteristics, by using the strain Td50b as a composting activator.

Further the invention will be described in details in the following examples.

*Example 1*. The strain of *Trichoderma harzianum* Rifai, strain Td50b, was isolated from a sample of fir bark compost, originating from Stefaneşti, Argeş, Romania (latitude, 44° 51' 53.49"N; longitude 24° 57' 0.67"E). The isolates were obtained by using the selective media proposed by William et al., 2003 (Appl. Environ. Microbiol., 69: 4190-4191), which contain 0.2 g $MgSO_4.7H_2O$, 0.9 g $K_2HPO_4$, 1.0 g $NH_4NO_3$, 0.15 g KCl, 0.15 g Rose Bengal (sodium salt, R3877 Sigma, Sigma - Aldrich, Saint Louis, MO, SUA), 3 g glucose, 20 g agar in 950 ml distilled water, on which was added after sterilization (by autoclaving at 121°C for 20 min) the ingredients which confer selectivity. The used antimicrobial ingredients, which confer selectivity to the media were (expressed per liter of media): 0.25 g chloramphenicol (C0378 Sigma - Aldrich), 9 ml of stock solution of streptomycin (1% weight/volume, S6501 Sigma-Aldrich), 0.2 g pentachloronitrobenzen (quintozene, P2205 Sigma-Aldrich) and 1.5 ml formulated propamocarb (Previcur 607 SL, Bayer Crop Science, Monheim am Rhein, Germany, 607 g active ingredient per liter). All these ingredients were brought to 40 ml with sterile distilled water, were sterilized by filtration and then added to the basal media cooled at the limit of solidification.

Selection of the strain Td50b from the isolates obtained on the above described selective media was done according to the bellow described process.

In a 24 well plate (Corning® Costar® cell culture plates, 24 well, flat bottom, Corning, Tewksburry, MA, USA) were aseptically distributed media which allow also revealing the activity of endo-cellulases. These agarized media used for the identification of cellulolysis consist of a basal media (1 liter of basal media contain 5 g of ammonium tartrate, 1 g $KH_2PO_4$, 0.5 g $MgSO_4 \cdot 7H_2O$, 0.1 g yeast extract, 0.001 g $CaCl_2.2H_2O$ - 0.1 ml from a stock solution 10 mg/ml), on which were added 2% carboxymethylcellulose of low viscosity (CMC 100 cP), and which was agarized with 20 g/l agar. The media was autoclaved at 121°C for 20 min.

The wells with agarized media were inoculated with 5 strains (4 isolated and one standard strain, *Trichoderma atroviride* ATCC 74058, for which was revealed the production of 6-pentyl-pirone, Reithner et al., 2005, Fungal Genet. Biol., 42:749-760) in 4 repetitions. It was done also a control, with agarized media, which was not inoculated. The randomization schedule for the 24 wells plate, 6 treatments (V1-control, inoculated, V2 - standard strain; V3-V5 - isolates for testing) in 4 repetitions, used during the screening experiment, is presented in tab. 1.

Tab.1. Randomization schedule for the 6 treatments (stains and controls) in 4 repetitions applied on the 24 wells plate.

|   | 1 | 2 | 3 | 4 | 5 | 6 |
|---|---|---|---|---|---|---|
| A | V4 | V5 | V3 | V6 | V2 | V1 |
| B | V6 | V2 | V4 | V5 | V1 | V3 |
| C | V3 | V1 | V5 | V2 | V4 | V6 |
| D | V5 | V3 | V1 | V4 | V6 | V2 |

The plate was covered with a sterile plastic film (Breathe Easy™ sealing membranes, Z380059 Sigma), which allow the exchange of gases, and incubated for 3 days at 25°C. Colony grown was checked visually. It was grown alongside, for 2 days, the test phytopathogen *Rhizoctonia solanii* 66873 ATCC (American Type Culture Collection, Manassas, VA, USA, isolated from a coniferous bark mulch, belonging to the hyphal anastomosis group AG-4, group which predominantly found as the causative agent producing diseases on ornamental plants) on a 24 wells plate, each containing 1.5 ml of potato dextrose agar (PDA) medium, inoculated with 0.1 ml of R. *solanii* hyphal suspension, containing 2.5 mg hyphae per ml (reconstituted suspension from mycelium grown on potato-glucose liquid medium). After 3 days of *Trichoderma* colony development, it was deposited the well plate, with agarized media on which *Trichoderma* tested strains emerged, over the one that contains the test microscopic fungus. Between the two plates was maintained the sterile film, which allow the exchange of gases. The joint plates, with media of grown *Trichoderma* isolates over the plate with phytopathogen test fungus, separated by the plastic film that allows the exchange of gases, were incubated for 7 days at 24°C. Development of test phytopathogenic fungus was determined by analyzing the well plate images, taken with an InGenius photodocumentation system and processed by the software Dimension (Syngene, Cambridge, United Kingdom). The percentage of inhibition was calculated using the formula (1) below:

$$\frac{A_1 - A_n}{A_1 x 100} \tag{1}$$

where $A_1$ is the surface covered by the phytopathogen agent in the untreated control, and An is the surface covered by the phytopathogen agent on the treatment wherein it was confronted with volatile compounds produced by one of the tested isolates. The data related to the inhibition of the fungus-test were processed by analysis of variance (Statistica, StatSoft, Tulsa, OK, USA), the inhibition activity of tested isolates being determined in comparison with the standard strain.

The agarized media, on which *Trichoderma* isolates grew, were flooded with a solution of 0.1% Congo Red, and, after 5 min, Congo Red solution was discarded and the plates were washed with a 1 M NaCl solution for 20 minutes. The strains of *Trichoderma* producing cellulases were revealed by identification of the areas of clear cellulolysis around the colonies.

After more than 25 cycles of testing, during which were tested over 100 isolates, the strain Td50b was identified, as the one which present, in the same time, a significant cellulolytic activity, highlighted by clear zones around colonies, and high inhibition activity due to the volatile compounds on test-fungus.

Taxonomic classification of *Trichoderma harzianum* Td50b is: *Ascomycota* Phylum, *Sordariomycetes* Class, *Hypocreales* Order, *Hypocreaceae* Family, Trichoderma genus.

Morphological characteristics of strain Td50b are described below.

The development of the colony: 4.5-7.5 (-9.0) cm in diameter, after 5 days, on PDA medium, initial $\pm$ hyaline, whitish-green, with areas of bunches of blue-green conidiophores; reverse of the colony without color;

Conidiophores: branching pyramidal, with shorter branches towards the apex;

Phialides: in groups of 2-4, quite thin and often curved, (6) 8-14 (-20) X 2.4 - 3.0 μm;

Conidia, subglobose to broadly ellipsoidal, 3.6-4.5 μm in diameter, with rough walls;

Chlamydospores present in the mycelium of older cultures, interlaced and sometimes terminal, most often globose, hyaline, and with smooth walls.

Physiological characteristics, of using different growing substrates, are described below.

Carbon sources: *optimal:* mannnite, fructose, ribose, glucose (dextrose), galactose, mannose; *moderate fungal development* on: arabinose, sorbose, melibiose, maltose, lactose, ceiiobiose, cellulose, starch, inulin; *fungal poor development* on: sorbitol, xylose, sucrose, glycerol;

Nitrogen sources: *optimal:* DL-leucine, L-citrulline, DL-cysteine, DL-norleucine, ammonium nitrate, ammonium tartrate; *fungal development moderate:* L-arginine, L-leucine, glycocol, asparagine, riboflavin, ammonium sulfate, ammonium carbonate, ammonium phosphate monobasic; *fungal poor development* on: tryptophan, tyrosine, D-serine, lysine, urea, sodium/calcium/potassium nitrates.

Physical characteristics of growth and sporulation are:

Temperature: *optimal temperature:* 20-25°C; *minimum temperature:* 2°C;
*maximum temperature:* 37°C;

The reaction of the substrate of culture: *optimal pH:* 4.0-5.5; poor development of the values of pH from 9.0 to 13.0.

Identification done on morphological criteria was confirmed by molecular analysis of ITS1 (internal transcribed spacers 1) of cluster rRNA gene (fungal universal marker BarCode, http://www.isth.info). Were used the specific primers ITS1 SR6R f and LR1 r (according to BarCode protocol http://www.isth.info/method) Nucleotide sequencing was done with the method *Dye Terminator Cycle Sequencing,* using an automat DNA sequencer model ABI PRISM 310 (Perkin Elmer. Waltham, MA, USA). Sequences comparison was done with the programme TrichoBlast (http://www.isth.info/tools/blast/index.php).

Td50b is a strongly antagonistic strain against phytopathogenic fungi, *Rhizoctonia solanii, Fusarium graminearum, F. culmorum, Pythium ultimum, Botrytis cinerea, Alternaria alternata, Sclerotinia sclerotiorum.* The degree of antagonism of the strain *Trichoderma harzianum* Td50b against phytopathogenic fungi was determined *in vitro* by the method of double confrontation assay (Coskuntuna and Özer, 2008, Crop Protection, 27: 330-336). The strain of *Trichoderma harzianum* Td50b and the phytopathogen agents (*Rhizoctonia solanii* ATCC 66873, *Fusarium graminearum* DSM4527, *F. culmorum* ATCC 36017, *Pythium ultimum* DSM 62987, *Botrytis cinerea* DSM 5144, *Alternaria altemata* DSM 62010, *Sclerotinia sclerotiorum* DSM 1946) were grown separately on medium potato glucose agar (PDA), for 7 days at 25°C and in the dark. From the lawn formed after 7 days it have been taken, with a punch, 0.5 mm round agar plugs, which were deposited at 1 cm from the walls of a Petri dish of 10 cm diameter, in opposite sides, each agar plugs being separated by approx. 6 cm. Interaction between colonies was determined after 5 days of incubation at 25°C and in the dark, using the scale proposed by Bell and co-workers (Bell et al., 1982, Phytopathology, 72: 379-382), with 5 notes: 1 - *Trichoderma* strain grew completely over phytopathogen, covering the entire surface; 2 - *Trichoderma* strain grew more than two-thirds on the surface of the culture medium; 3 - *Trichoderma* strain and pathogen grew each on about half of the area of the agar medium; 4 - phytopathogen grew on more than two-thirds of the surface of the culture medium; 5 - phytopathogen grew completely over *Trichoderma,* covering the entire surface. Experiments were performed in triplicate and repeated three times.

From the analysis of the experimental results obtained *in vitro* (table 2), it is found that the strain of *Trichoderma harzianum* Td50b presents a pronounced antagonism to *Rhizoctonia solanii* ATCC 66873, *Fusarium graminearum* DSM4527 *f. culmorum* ATCC 36017, *Pythium ultimum,* DSM 62987, *Botrytis cinerea* DSM 5144, *Alternaria alternata* DSM 62010, *Sclerotinia sclerotiorum* DSM 1946.

Tab. 2. *In vitro* antagonism of the strain of *Trichoderma harzianum* Td50b against different phytopathogen agents.

| Phytopathogen agent | Antagonistic activity | Reaction |
| --- | --- | --- |
| *Rhizoctonia solanii ATCC* 66873 | 1.77 | Strong antagonist (SA) |
| *Fusarium graminearum* DSM4527 | 1.88 | Strong antagonist (SA) |

(continued)

| Phytopathogen agent | Antagonistic activity | Reaction |
|---|---|---|
| *F. culmorum* ATCC 36017 | 1.88 | Strong antagonist (SA) |
| *Pythium ultimum* The DSM 62987 | 1.66 | Strong antagonist (SA) |
| *Botrytis cinerea* DSM 5144 | 1.44 | Strong antagonist (SA) |
| *Alternaria alternata* DSM 62010 | 2.00 | Strong antagonist (SA) |
| *Sclerotinia sclerotiorum* DSM 1946 | 1.88 | Strong antagonist (SA) |

It was established also the activity of the enzyme from the mycelium of *T. harzianum* Td50b, obtained after growing on a basal liquid medium, supplemented with 2% CMC. 1 liter of basal medium contains: 5 g ammonium tartrate, 1 g $KH_2PO_4$, 0.5 g $MgSO_4.7H_2O$, 0.1 g yeast extract, 0.001 g $CaCl_2.2H_2O$ - 0.1 ml of stock solution 10 mg/ml, to which has been added 2% of low-viscosity carboxymethylcellulose (CMC 100 cP). On this medium, sterilized by autoclavation 20 min at 121 °C, was cultivated *T. harzianum* Td50b for 7 days. It was collected about 1 g of the mycelium, which was homogenized in 100 mM TRIS buffer (pH 7.5) at 4°C for one four, using a Thermomixer (Eppendorf, Hamburg, Germany). The supernatant was recovered after centrifugation at 10,000xg at 4°C for 20 min. The total protein in extract was assayed by Bradford method, using bovine serum albumin as standard. The enzymatic activity was determined using filter paper Whatman No. 1 as substrate. Filter paper has been cut into strips 1 x 6 cm, which weight 50 mg; these were suspended in a 18 mm/10 ml test tube, in 1 ml of citrate - sodium buffer 0.05 M, pH 4.8. Over the mixture filter paper - buffer were added 0.5 ml of enzymatic extract. It was vortexed, for an efficient maintenance in suspension of the filter paper, and incubated for 1 h at 45°C. After the incubation period the reaction was stopped with 3 ml dinitrosalicilic reagent (DNS, containing 283.2 ml deionized water, 2.12 g 3,5-dinitrosalicilic acid, 3.96 g NaOH, 61.2 g double sodium and potassium tartrate, 1.52 ml liquid phenol 89% and 1.66 g sodium bisulfite). The tube was heated on boiling water bath for 5 min, for the development of the color reaction, after which it was read the optical density at 545 nm. Enzyme activity was expressed in units of the FPU (Ghose, 1987, Pure Appl. Chem, 59: 257-268), using a standard curve of glucose. 1 unit FPU is 0.37 / enzyme activity releasing the equivalent to 2 mg of glucose enzyme units ($\mu$moles of reducing groups which reacts with DNS, released per min). Strain Td50b produced in the given experimental conditions 0.32 $\pm$ 0.08 FPU per mg protein, which means it is a high cellulase producing strain. (Kovacs et al., 2008, Enz. Microbiol., Technol., 43: 48-55).

It was also determined the ability of Td50b to produce and accumulate 6-pentyl-$\alpha$-pyrone (6-PP.) Strain Td50b and ATCC 74058 were grown on liquid medium potato-dextrose, stationary, without stirring, for 5 days at 25°C. After 5 days the mycelium formed was homogenized within culture medium by using a blender (Waring®, Laboratory Blender, Fischer Scientific, Waltham, MA, USA). The amount of biomass in the homogenate was determined gravimetrically after filtration on filter paper Whatman No. 1 and drying at 105°C to constant mass. 25 ml of homogenate biomass - culture medium were taken and were extracted with 25 ml of methylene chloride. Inferior lawyers, containing the solvent with density greater than that of the water, were reunited, dried over anhydrous sodium sulfate, concentrated to dryness and re-solubilized in 0.1 ml methylene chloride. Determination of 6-PP was made by gas-chromatography, with mass spectrometer detector. It was used an Agilent 700 gas chromatograph, equipped with quadrupol mass spectrometer (Agilent, Santa Clara, CA, USA). 6-PP was separated on a DB-5 column (0.32 mm internal diameter, length 30 m, 0.25 $\mu$m film thickness). It was applied 1 $\mu$l sample, in the split mode, with a split ratio of 1:100 till the end of the period of separation. The column was maintained at 40°C for 2 min, followed by a gradient of 20°C/min up to 120°C, which was maintained for 2 min, and then increased to 210°C with 10°C/min. Temperatures of detector and injector were 250°C. Helium was used as carrier gas, with a flow rate of 1 ml/min. For quantification it was done a standard curve, using pure 6-PP (Sigma-Aldrich). The determined 6-PP quantity was related to the volume of culture medium and to the amount of dry biomass determined gravimetrically in the culture medium. The experiments were done in triplicate. The results are presented in table 3, as average of the three repetitions, compared with data reported for other strains of *Trichoderma* grown on liquid medium. These results support the affirmation related to ability of Td50b to synthesize significant amounts of 6-PP.

Tab. 3. Efficiency of 6-pentyl-$\alpha$-pyrone synthesis by the strains tested, compared with those reported for other strains grown on liquid medium.

| Strain | Growth conditions | Final concentration | Reference |
|---|---|---|---|
| *T. harzianum* Td50b | Potato-dextrose, 25°C, 5 days | 350 mg/l 83,41 mg/h | This example |

(continued)

| Strain | Growth conditions | Final concentration | Reference |
|---|---|---|---|
| *T. atroviride* ATCC 74058 | Potato-dextrose, 25°C, 5 days | 238 mg/l 65,75 mg/g | This example |
| *T. harzianum* IMI 206040 | Malt extract - glucose, 29°C, 5 days | 182 mg/l 60,67 mg/g | Serrano-Carreón *et al.,* 2004[1] |
| *T. harzianum,* Indian strain | Potato-dextrose, 30°C, 5 days | 455 mg/l 98,91 mg/g | Kalyani *et al.,* 2000[2] |
| *T. konigii,* IMI 308475 | Potato-dextrose, 20°C, 5 days | 145 | Simon *et al.,* 1988[3] |
| [1]-Serrano-Carreón et al., 2004, Biotechnol. Lett., 26: 1403-1406<br>[2]-Kalyani et al., 2000, Appl. Microbiol. Biotechnol., 53: 610-612<br>[3]-Simon et al., 1988, Soil Biol Biochem., 20: 263-264 | | | |

*Example 2.* Strain *T. harzianum* Td50b was tested in terms of antagonism *in situ,* on the residue of roses, against *Botrytis cinerea* DSM 5144 (isolated originally from roses). Discs with a diameter of 1 cm of green leaves and petals of rose (*Rosa hybrida* cv. Sonia), were taken and inoculated with 0.3 ml of the spore suspension $10^6$ conidia/ml of *B. cinerea* DSM 5144, applied by spraying with a glass sprayer with metal top and plastisol bulb (model 15-RD, DeVilbiss Healthcare, Somerset, PA, USA). Inoculated disks were placed in the rolls of paper towels moistened, and rolls of paper towels were placed inside of transparent polyethylene bags. The bags were sealed and kept at room temperature, for 3 days in the case of the petals, and for 7 days in the case of leaves, in order to develop the characteristic symptoms of infection with *B. cinerea.*

The disks of petals and leaves with symptoms of gray mold were then left to dry for 10 days, to simulate residues of dried roses infected with *Botrytis cinerea.* Infected disks and dried were then immersed for 20 seconds in a suspensions of spores of *T. harzianum* Td50b in sterile phosphate buffer saline (PBS), $10^4$ and $10^5$ spores/ml, respectively, and in sterile phosphate buffer saline as a control, and then these were distributed in 12 Petri dishes with a diameter of 15 cm. Experiment included the following treatments:

$V_1$ - control, inoculated with *B. cinerea* and un-treated
$V_2$ - control, inoculated with *B. cinerea* and subsequently treated with sterile PBS
$V_3$ -inoculated with *B. cinerea* and treated with *T. harzianum* Td50b $10^4$ spores/ml
$V_4$ -inoculated with *B. cinerea* and treated with *T. harzianum* Td50b $10^5$ spores/ml

Each treatment was done in 4 repetitions, each repetition being represented by the 12 discs of petals and leaves, arranged in Petri dishes of 15 cm. Petri dishes with various repetition were random placed during the experimental procedures.

Petri dishes were kept for 24 hours in darkness at 25°C in a humid chamber (Model HCP108, Scwabach, Memmert, Germany) and then were moved in a climatic chamber (Model GSC 120 LED, Weiss Gallenkamp, Lougborough, United Kingdom), where they were maintained at 60 $\mu$mol photons/m$^2$/s, with a 12 hours photoperiod, 20 ° C and 70% humidity, 3 days for petals and 7 days for the leaves.

After incubation, it was estimated the surface on which were formed conidiophores of *B. cinerea,* by dissecting and examination under a binocular microscope, using the following grading scale: 0 - less than 1 %; 1 - between 1 and 25%; 2 - between 25 and 50%; 3 - between 50 and 75%; 4 - between 75 and 99%; 5 over 99%. Average intensity of sporulation was calculated as a weighted average for each repetition, according to the formula (2) below.

$$I = \frac{\sum_{i=1}^{n} n_i p_i}{\sum_{i=1}^{n} p_i} \qquad (2)$$

where: $n_i$ are note in accordance with the scale from above, and $p_i$ *are* percentage weight from the total notes of each note.

The data were statistically processed by analysis of variance (Statistica, StatSoft).

The results of the experiment are presented in table 4. These results demonstrate a significant capacity of Td50b strain *of Trichoderma harzianum* to inhibit the sporulation of microscopic phytopathogenic fungus *B. cinerea* on rose plant debris.

Tab. 4. The influence of the treatment with spore suspension of *T. harzianum* Td50b on sporulation of phytopathogen B. *cinerea* on rose plant debris*.

| Treatment | Average intensity of sporulation on leaves | Average intensity of sporulation on petals |
|---|---|---|
| $V_1$ - control, inoculated with *B. cinerea* and un-treated | 3.25a | 4.12a |
| $V_2$ - control, inoculated with *B. cinerea* and subsequently treated with sterile PBS | 3.62a | 4.37a |
| $V_3$ -inoculated with *B. cinerea* and treated with *T. harzianum* Td50b $10^4$ spores/ml | 0.88b | 1.37b |
| $V_4$ -inoculated with *B. cinerea* and treated with *T. harzianum* Td50b $10^5$ spores/ml | 0.75a | 1.12a |
| DL5% | 0.88 | 0.75 |
| * Values followed by the same letter does not differ significantly P > 0.05 | | |

This pronounced antagonism *in situ* has a practical significance, the presence of antagonist Td50b reducing the formation of the primary inoculum of B. *cinerea* in infected plant debris of rose and, consequently, the development of gray mold disease of the rose.

*Example* 3. Strain *T. harzianum* Td50b was tested in terms of its antagonistic capacity *in vivo,* for protection of ornamental plants against phytopathogen agents. Seeds of hybrid geranium (*Pelargonium x hortorum*), "Moulin Rouge" were sown on April 18, to get stock plants, from which were obtained by cutting, in early September, the plants for experiments. The cuttings were place in a mist chamber, in vermiculite, to stimulate roots development. After three weeks uniform cuttings, in terms of root and shoot development, were planted in plastic pots of 15 cm, containing a growth substrate enriched with nutrients for the first weeks of growth (Canna Terra Professional Plus, Canna International BV, Oosterhout, Netherlands). Flower pots were kept in greenhouse conditions at $22 \pm 2°C$ during the day and $17 \pm 2° C$ during the night, with a 12 hours photoperiod, supplemented with light intensity of 160 mcE/m²/s, derived from halogen lamps when light intensity decrease below 500 mcE/m²/s. Substrate contained an initial nutrient reserve and consequently the plants were fertilized only after three weeks of growth by applying 100 ml of nutrient solution 1 g/l of fertilizer 20-8-20 (N-$P_2O_5$-$K_2O$ Eurofertil, TimacAgro, Romania).

This *in vitro* testing experiment included the following treatments:

$V_1$ - control un-inoculated (with phytopathogen or Td50b);
$V_2$ - inoculated with *T. harzianum* Td50b, $10^4$ spores/ml, 4 weeks after transplantation;
$V_3$ - inoculated with *Pythium ultimum,* DSM 62987, $10^6$ propagules/ml, 8 weeks after transplantation;
$V_4$ - inoculated with *T. harzianum* Td50b, $10^4$ spores/ml at 4 weeks after transplanting and *Pythium ultimum,* DSM 62987, $10^6$ propagules/ml, 8 weeks after transplantation.

Each treatment included 12 pots, which were arranged in blocks of three per repetition, in a Latin square random schedule, 4 variations in 4 repetitions.

At 4 weeks after cuttings transplantation the soil from the treatments $V_2$ and $V_4$ was inoculated with 50 ml of sterile phosphate saline buffer, 0.1 M, pH 7.2, containing $10^4$ cfu/ml *T. harzianum* Td50b. The antagonistic strain was grown on Roux bottles, containing potato-glucose-agar medium, for 7 days. Spores formed were recovered in a sterile phosphate buffer, 0.1 M, pH 7.2. Propagules number normalization was done by using a microscopic counting chamber. On the others two treatments, $V_1$ and $V_2$, were applied 50 ml of sterile phosphate saline buffer, 0.1 M, pH 7.2.

At 8 weeks, after cuttings transplantation, were inoculated the experimental treatments $V_3$ and $V_4$ with *Pythium ultimum.* Because at the room temperature P. *ultimum* do not produce zoospores (van der Plaats-Niterink, 1981, Monograph of the genus Pythium. Institute of the Royal Netherlands Academy of Sciences and Letters, Baarn, Netherlands, pp. 164-167), the suspension of propagules of *P. ultimum* was obtained from hyphae and oospores, being prepared by cultivation on liquid medium (250 ml erlenmeyer flasks), which contained 100 ml of potato glucose liquid medium, incubated on a rotary shaker, at 150 revolutions per minute, for 7 days, at 24°C. Propagules number was determined by using a microscopic counting chamber, being brought to $10^6$ cfu/ml in a potato glucose liquid medium.

At 4 weeks after the infection with *P. ultimum,* and 12 weeks after the transplantation, the fresh mass and the dry mass of roots and aerial parts (shoots and leaves) of geranium plant were weighed. The data were statistically processed by analysis of variance (Statistica, StatSoft).

The results are presented in table 5 and demonstrate both the existence of an antagonism of *Trichoderma harzianum* strain Td50b against *P. ultimun,* and the capacity of this strains to stimulate geranium plants growth, in the given experimental conditions.

Tab. 5. Effect of treatments with *T. harzianum* Td50b on the development of the disease caused by *P. ultimum* on geranium and on geranium plant growth*.

| Experimental treatment | Root mass, g | | Shoot mass, g | |
|---|---|---|---|---|
| | fresh | dry | fresh | dry |
| $V_1$ - control un-inoculated (phytopathogen or Td50b) | 13.04 | 1.74b | 107.16 c | 14.83bc |
| $V_2$- *T. harzianum* Td50b, $10^4$ spores/ml | 16.24a | 2. 85a | 127.42a | 17.12a |
| $V_3$- *P. ultimum* DSM 62987, $10^6$ /ml propagule | 10.62c | 1.25 c | 105.74c | 14.24c |
| $V_4$- *T. harzianum* Td50b, $10^4$ spores/ml *P. ultimum,* DSM 62987, $10^6$ /ml propagule | 14.08ab | 2.34a | 116.41 b | 16.35ab |
| DL 5% | 1.72 | 0.35 | 9.24 | 1.07 |
| * Values followed by the same letter does not differ significantly P > 0.05 | | | | |

Another experiment had the goal to determine the ability of Td50b to protect the seedlings of ornamental plants against damping-off produced by *Rhizoctonia solanii.*

The inoculum of *Rhizoctonia solanii* ATCC 66873, anastomosis group AG4, was grown on wheat bran for 10 days. Wheat bran was sieved in order to obtain the fraction 0.25 ... 0.5 mm, washed with distilled water, distributed in Roux bottles and sterilized, repeatedly - twice, by autoclavation at 121 °C for 30 min. Brans were distributed by 100 g in 450 ml Roux plates, moistened with 50 ml of sterile phosphate saline buffer, 0.1 M, pH 7.2 and inoculated with 1 ml suspension $10^6$ propagules /ml, obtained from cultures grown on agarized media.

The used growth substrate for ornamental plants was Potground H70 substrate (Klasmann-Delimann, Geeste, Germany), pH 6.0, containing 25% white peat 0-7 mm, 75% dark peat and 1.5 g/l complex NPK fertilizer, which is specially designed for production of vegetables and flowers seedlings.

The growth of ornamental flowers was done in plastic punnets with 32 trays (Model EPE 32, Marcoser, Matca, Galati, Romania), each seedling tray having a volume of 102 ml.

Ornamental plants used for the experiment were pansies (*Viola x wittrockiana* cv. Bern), petunia (*Petunia x hybrida* cv. Colour parade F1), cockscomb (*Celosia cristata,* cv. Orange).

Growth substrate was mixed with infected bran, in a ratio of 0.25% v/v; 7 ml of this inoculated mixture was put at the bottom of each seedling tray, and over this substrate infected with *Rhizoctonia solanii* were deposited 68 ml of grown substrate, sterilized by gamma irradiation. In the case of the inoculated control the growth substrate was mixed with wheat bran sterilized by autoclavation and moistened with sterile phosphate buffer, 0.1 M, pH 7.2.

Treatments against damping-off were performed with a suspension of *T. harzianum* Td50b and with a standard chemical product, formulated propamocarb (Previcur 607 SL, Bayer Crop Science, 607 g active ingredient per liter). Strain Td50b was applied as suspension, $10^4$ spores in sterile phosphate buffer saline, 0.1 M, pH 7.2, 3.25 ml for each tray (corresponding to a dose of $10^3$ spores/g of substrate). Formulated propamocarb was diluted to 1 % in deionized water, and from this solution were applied 3 ml per each tray having a diameter 6.2 cm, corresponding to a recommended dose of 10 ml of formulated product per square meter of seedling soil. Un-inoculated control and the control untreated with products against damping-off were treated with 3.25 ml sterile phosphate buffer saline, 0.1 M, pH 7.2.

On each tray were introduced seeds of ornamental plants already specified. Seedlings were maintained in the greenhouse conditions at $22 \pm 2$°C during the day and $17\pm2$°C during the night, with 12 hours photoperiod, supplemented with light intensity of 160 mcE/m$^2$/s, produced by a halide lamps when light intensity decrease below 500 mcE/m$^2$/s. Growth substrate contained initial nutrient reserve and consequently the plants were fertilized only after two weeks of growth, by applying 100 ml of nutrient solution 1 g/l of fertilizer 20-8-20 (N-P$_2$O$_5$-K$_2$O TimacAgro Eurofertil, Romania).

Each treatment, $V_1$ - un-inoculated control, $V_2$ - infested with R. *solanii* and untreated with products against damping-off, $V_3$ - chemical product (standard) treatment; $V_4$ - treatment with *T. harzianum* Td50b, was carried out in 4 repetitions, each repetition being represented by a plastic punnet. Punnets with seedlings were arranged in a randomized biock in greenhouse, host plants being randomized between blocks, and different treatments in the frame of host plant sub-blocks.

At 3 weeks after sowing the survival rate of seedlings of ornamentals plants was determined. The obtained data were statistically processed by analysis of variance (Statistica, StatSoft).

The results are presented in table 6 and show a high efficacy of Td50b in the protection against damping-off caused by R. *solanii* AG-4, similar to that of the chemical (standard) product.

Tab. 6. Influence of treatment with *t. harzianum* Td50 on survival of plantlets of ornamental plants grown on a substrate inoculated with *r. solanii Ag-4*

| Experimental treatment | Average number of plants per punnet with 32 trays: | | |
|---|---|---|---|
| | *Viola x wittrockiana* | *Petunia x hybrida* | *Celosia cristata* |
| Control, un-inoculated with *R. solanii* AG-4 and untreated | 24.25 | 25.75 | 25.5 |
| Control, inoculated with *R. solanii* AG-4 and untreated | 4.0 | 6.5 | 9.25 |
| Propamocarb, equiv. 10 ml f./p. per m$^2$ | 19.5 | 20,25 | 22.5 |
| *T. harzianum* Td50b, 10$^3$ spores/g substrate | 18.5 | 20.5 | 20.25 |
| DL5% | 8.25 | 7.5 | 7.5 |

*Example 4.* Strain *T. harzianum* Td50b was multiplied and included in a composition which promote its colonization on compostation substrate. Multiplication was done on an industrial media based on whey. In the sweet whey obtained from curd manufacture the dry matter was determined by refractometry, was diluted to 3% dry matter from whey, with deionized water, and then were added 1.5% corn distiller grains from ethanol production (mass / volume); 0.5% $KH_2PO_4$, (mass / volume); 0.5% $(NH_4)_2SO_4$ (mass / volume). 100 ml of this mixture were distributed in 500 ml erlenmeyer flasks, sealed with cotton wool, and sterilized at 121 °C for 25 min; after that were inoculated aseptically with 5 ml of suspension containing 10$^7$ spores/ml and incubated for 5 days at 25°C on the rotating shake, 150 rpm. 10 ml from this suspension were used to inoculate polypropylene bags that contained 100 grams fir sawdust, wetted with 50 ml deionized water. Polypropylene bags with sawdust wetted were previously sterilized at 121°C for 25 min.

After inoculation the bags were incubated for 7 days climatic chamber (Model GSC 120 LED, Weiss Gallenkamp, Lougborough, UK), where these were maintained at 60 $\mu$mol photons/m$^2$/s, with 12 hours photoperiod, at 25°C and 70% humidity. After 7 days the bags were irradiated for 15 min with blue light 440-460 nm, 60 $\mu$mol photons/m$^2$ /s, and then the bags were incubated in the dark, at 25°C, for another three days.

Biomass of *T. harzianum* mixed with sawdust partially degraded was mixed in the ratio of 1:1 with a suspension containing 10% wheat flour, 10% mixture of ethyl esters of fatty acids, lecithin, potassium soaps, glycerol, lipids from rapeseed oil and 4% polyvinyl alcohol (26-88, EMPROVE® powder, Merck, Darmstadt, Germany). The resulting paste was extruded on a pasta machine (Model TR95A, Helco, Craiova, Romania), and the resulting noodles were granulated on a spheronization equipment (model Spheronis-250 m Grabler, Ettlingen, Germany). The resulted granules were dried in a fluid bed dryer (Model TC20, Retsch, Germany), at a maximum temperature of 37°C.

The mixture of ethyl esters of fatty acids, lecithin, potassium soap, glycerol, unsaponifiable lipids from rapeseed oil, was obtained according to the procedure described below. 1 000 g of degummed rapeseed oil, with the characteristics shown in table 7, was brought-up in a 2 liters stainless steel autoclave, with stirring and heating, under protective atmosphere of nitrogen.

Tab. 7. Characteristics of degummed rapeseed oil used

| Water and volatile compounds | % m/m | 0,4 |
|---|---|---|
| Unsaponifiable substances | % m/m | 1.4 |
| Free fatty acids | % m/m | 1.9 |
| Saponification number | mg KOH/g | 169,5 |
| The average fatty acid composition (% w/w): C16: 2.4; C18: 2; C18-1: 10.0; C18-2: 24.5; C18-3: 7.3; C20-1: 4.5; C22-1: 42.4 | | |

25 g of potassium hydroxide purity 98% were dissolved on 210 g of ethanol, 0.3% water and the resulting solution was posted in autoclaves over degummed rapeseed oil. It was started the agitation and the heating to 40°C. After a reaction time of 8 hours, the reaction mass was cooled to room temperature. It was collected 1225 g transparent reaction mass (R1). 500 g of R1 was treated with oleic acid, yielding a product (P1) having the following composition: (% w/w): fatty acid ethyl esters (FAEE) 74.5; triglycerides 5.9; glycerol 1; potassium soap 11.4 and water 1.1. 140 g of the product of the reaction (P1) was treated under vigorous shaking with 60 g soy lecithin liquid emulsifiant, resulting a mixture with the following composition: (% w/w): fatty acid ethyl esters (FAEE) 48.7; not reacted fats from rapeseed oil 4; glycerol 3; potassium soap 7.5, lecithin 34.6 and water 0.7. This mixture was used to obtain the controlled-release composition containing *T. harzianum* Td50b.

Controlled-release composition based on *T. harzianum* Td50b resulted is made of 70 parts sawdust partially hydrolyzed, 10 parts flour, 4.8 parts ethyl esters of fatty acids, 4 parts polyvinyl alcohol, 3.5 parts of lecithin, 0.75 parts of potassium soap, 0.45 parts glycerol, 0.4 parts lipids from rapeseed oil, the difference till 100 parts being water, and min. $10^6$ ufc/g *T. harzianum* Td50b.

The number of spores of *T. harzianum* Td50b from the above composition was determined by extraction with phosphate buffer saline, serial dilutions and cultivation on the selective medium proposed by William *et al.,* 2003, presented in detail in Ex.1.

The composition was tested in terms of its action on the acceleration of plant material degradation. Plant material (fir tree bark) was milled and passed on the sieve of 0.250 mm. Form the resulting ground powder were taken 10 g, which were brought to a 50 ml erlenmayer flask, together with 20 ml of distilled water. The mixture was homogenized by vigorous shaking and then was inoculated with 1 ml of suspension of T. *harzianum* Td50b containing $10^6$ spores/ml, or 1 gram of composition according to this example. All experiments were done in 5 repetitions and erlenmeyer flasks were kept at room temperature for 7 days.

After incubation, the mixture was passed into a respiration vessel Strathox (Strathkelvin Instruments Limited, Glasgow, United Kingdom). There were done in parallel determinations of respiration / oxygen consumption for 12 hours.

After finishing the determinations of respiration, samples of 1 g material were taken and these were extracted with 1 ml of methylene chloride for 20 min. 0.45 ml of extract were placed in a single phase filtration vial, with 0.2 $\mu$m nylon membrane filter (Thomson Instrument Company, Oceanside, CA, USA), and in the filtrate was determined 6-PP by gas chromatography, in accordance with the method presented already.

The samples were analyzed in comparison with a control without inoculum of *Trichoderma,* which was only treated with sterile water and incubated at room temperature under the same experimental conditions. The data were statistically processed by analysis of variance (Statistica, StatSoft).

The results are shown in tab. 8. These results demonstrate a high capacity of degradation of plant material by strain Td50b, which is significantly influenced by the inclusion of this strain in the composition obtained in accordance with this example.

Tab. 8. Degradation of fir tree bark by T. *harzianum* Td50b and accumulation of 6-$\alpha$-pentyl-pyrone in composted media*.

| Variant | Breathing (mg/l a$_2$ drink, hourly) | 6-PP (mg/g material) |
|---|---|---|
| *T. harzianum* Td50b suspension $10^6$ spores/ml | $1.06 \pm 0.32$b | $152 \pm 23$b |
| Composition Ex 4 with *T. harzianum* Td50b $10^6$ spores/g | $1.68 \pm 0.22$a | $273\pm32$a |
| Untreated with *Trichoderma* | $0.52 \pm 0.14$ c | ND |
| *Values followed by the same letter does not differ significantly P > 0.05. ND-undetectable | | |

*Example 5.* Composition obtained from example 4 from above was used to obtain suppressive compost, and the resulting compost was tested from the point of view of protecting ornamentals plant against diseases caused by phytopathogen agents. 10 kg of coarse crushed fir bark were put in plastic pots of 30 liters and were treated with 100 ml of *T. harzianum* Td50b suspension $10^6$ spores/ml or composition according to Ex. 4, containing *T. harzianum* Td50b $10^6$ spores/g. The substrate was wetted with 10 liters of solution 2% (w/v) of NP complex fertilizer 20-20 in tap water, containing 4 g N and 4 g P$_2$O$_5$, and homogenized. The experiment was done with a control treated only with 100 ml sterile water, every experimental treatments being done in four repetitions. The compost was maintained at room temperature for 90 days, in the days 22th, 44th and 66th from the beginning of the experiment being done analyses of the content of cellulose, lignin and 6-PP. The cellulose was determined after Soxhlet lipid extraction with cyclohexane, repeated treatments with an alkaline solution of sodium carbonate to remove hemicellulose, hydrolysis with sulfuric acid, sodium hydroxide neutralization and determination of reducing carbohydrates with DNS reagent. Lignin was determined gravimetrically from samples, after repeated reflux extractions with sulfuric acid solutions of defatted material. 6-PP was determined by gas-chromatography, after extraction in methylene chloride, as has already been presented.

All experiments were done in 4 repetitions, and the data were statistically processed by analysis of variance (Statistica, StatSoft). The results are presented in table 9 and demonstrate that treatment with strain Td50b accelerates the decomposition of plant material, and composition done according to invention promote the process of increased degradation of plant material. Levels of 6-PP stabilize to similar values in both cases of the treatments applied with *T. harzianum* strain Td50b, due to the volatility of this odorant compound.

Tab. 9. Acceleration of the degradation of tree bark substrate composted by *T. harzianum* strain Td50b and accumulation of 6-$\alpha$-pentyl pyrone in the material*.

| Treatment | Cellulose content (%) | | | Lignin content (%) | | | Content 6-PP (mg/g material) | | |
|---|---|---|---|---|---|---|---|---|---|
| | 22d | 44d | 66d | 22d | 44d | 66d | 22d | 44d | 66d |
| *T. harzianum* Td50b suspension $10^6$ spores/ml | 37.52 | 30.23 | 25.47 | 27.15 | 24.63 | 23.27 | 143 | 154 | 128 |
| Composition Ex. 4, *T. harzianum Td50b* $10^6$ spores/g | 35.61 | 28.74 | 23.12 | 26.03 | 24.02 | 22.83 | 181 | 173 | 148 |
| Untreated with *Trichoderma* | 39.62 | 33.51 | 28.72 | 28.22 | 26.17 | 24.43 | ND | 12 | ND |
| *Values followed by the same letter does not differ significantly P > 0.05. ND-undetectable | | | | | | | | | |

Compost products were used for the treatment of rose plants for protection against *Botrytis.* Plants of rose (*Rosa hybrida* cv. Sonia), were obtained by cutting, in pots with substrate H70 Potground (Klasmann-Delimann, Geeste, Germany), pH 6.0, which contains 25% white peat 0-7 mm, 75% dark peat and 1.5 g/l complex NPK fertilizer. The cuttings were rooted for 14 days in high humidity conditions, obtained by covering pots with plastic foils. After 5 weeks from planting date the plants were pruned with a garden scissors, at a height of 7 cm.

The plants were kept in a greenhouse, at $22\pm2°C$ during the day, and $17\pm2°C$ during the night, with 12 hours photoperiod, supplemented with a light intensity of 160 mcE/m$^2$/s, produced by halide lamps, when light intensity decrease below 500 mcE/m$^2$/s. The used substrate contained an initial nutrient reserves, and consequently the plants were fertilized only after one week from the end of the rooting period, by application of 50 ml of nutrient solution, 1 g/l of complex fertilizer 20-8-20 (N-$P_2O_5$-$K_2O$, Eurofertil, TimacAgro, Romania) on each pot.

After rooting the plants were divided into experimental blocks according to the below treatments:

$V_1$ - untreated, not-inoculated;

$V_2$ - untreated, inoculated with *B. cinerea* $10^6$ spores/ml;

$V_3$ - treated with chlorotalonil (standard product), inoculated with B. *cinerea* $10^6$ spores/ml;

$V_4$ - fir bark compost, un-treated with *Trichoderma,* applied as mulch after rooting; inoculated with *B. cinerea* $10^6$ spores/ml;

$V_5$ - fir bark compost, formed after treatment with *T. harzianum* Td50b suspension $10^6$ spores/ml, applied as mulch after rooting, inoculated with B. *cinerea* $10^6$ spores/ml;

$V_6$ - fir bark compost, formed after treatment with composition Ex. 4, containing *T. harzianum* Td50b $10^6$ spores/g, applied as mulch after rooting, inoculated with *B. cinerea* $10^6$ spores/ml.

Composts obtained from fir bark, with or without application of Td50b strain, were applied as mulch on the pot surface, thickness of about 2.5 cm, immediately after rooting of cuttings. The chemical treatment was done by coverage spraying, at pruning time and after 7 days, with a solution obtained by dilution of 2.5 ml of formulated product (Bravo 500 SC, Syngenta Crop Protection, Basel, Switzerland) in 1 liter of water. Inoculation with spores of *B. cinerea* was in the second day after pruning, with an previous increase of the humidity in the greenhouse at 80%. *Botrytis cinerea* DSM 5144 was cultivated on potato-dextrose-agar medium, in Petri dishes Ø 9 cm. Spore suspension was done by flooding the *Botrytis* lawn, grown for 10 days at 25°C, 70% humidity and 60 $\mu$mol photons/m$^2$/s, with a 12-hour photoperiod, with 5 ml of sterile phosphate buffer solution, containing Tween 20 0.04%, and the spores were released from the colonies with a Drigalsky spreader. The spore suspension was standardized to $10^6$ spores/ml by counting on the counting chamber. On each plant were sprayed 2 ml of suspension spores, using a glass sprayer with metal top and plastisol bulb (model 15-RD, DeVilbiss Healthcare).

The frequency of the attack on the leaves, the total number of leaves per plant and leaves wilted was determined at 2 weeks from the application of the inoculation with B. *cinerea.* All experiments were conducted in 4 repetitions, and the data were statistically processed by analysis of variance (Statistica, StatSoft) .

Table 10 shows the results obtained in this experiment. These results demonstrate that composts formed under the action of antagonistic *T. harzianum* Td50b forms compounds from the category of volatile odorants, which reduce the attack of *B. cinerea* on aerial plant parts, most likely through activation of the defense response of plants. Composts from for bark, formed under the action of antagonistic *T. harzianum* Td50b, release also nutrients and other biological active compounds that support and stimulate plant growth.

Tab. 10. Effect of different treatments, including through the application of compost of fir bark obtained with *T. harzianum* Td50b as mulch, on the frequency of the *B*. *cinerea attack,* the total number of leaves per plant and leaf wilting percentage.

| Experimental version | The attack frequency (%) | The total number of leaves per plant | Wilted leaf percentage (%) |
|---|---|---|---|
| Untreated not-inoculated control | $14.8 \pm 6.7$ab | $11.5 \pm 1.5$b | $21.53 \pm 1.52$a |
| *B. cinerea* $10^6$ spores/ml | $74.3 \pm 9.7$ c | $8.5 \pm 1.5$c | $69.04 \pm 2.38$d |
| Chlorotalonil (0.25% f.p.) *B. cinerea* $10^6$ spores/ml | $12.1 \pm 4.3$a | $13 \pm 2$ab | $19.09 \pm 0.91$a |
| Fir bark compost, *B. cinerea* $10^6$ spores/ml | $59.6 \pm 11.3$ c | $10.5 \pm 1.5$b | $49.94 \pm 1.52$c |
| Fir bark compost, formed after treatment with *T. harzianum* Td50b suspension $10^6$ spores/ml. *B. cinerea* $10^6$ spores/ml | $23.4 \pm 5.7$b | $12.5 \pm 2.5$ab | $28.33 \pm 1.66$b |
| Fir bark compost, formed after treatment with composition Ex. 4, containing *T. harzianum* Td50b $10^6$ spores/g, *B. cinerea* $10^6$ spores/ml | $16.5 \pm 5.8$ab | $14.5 \pm 1.5$a | $17.06 \pm 1.68$a |
| Values followed by the same letter does not differ significantly P > 0.05 | | | |

**Claims**

1. Strain of *Trichoderma harzianum* Td50b NCAIM (P) F 001412 **characterized in that** is antagonistic *in vitro* against *Rhizoctonia solanii, Fusarium graminearum, F. culmorum, Pythium ultimum, Botrytis cinerea, Alternaria alternata, Sclerotinia sclerotiorum;* protects the ornamental cultures against diseases produced by phytopathogenic fungi from soil involved in damping-off complex, *Rhizoctonia, Pythium, Fusarium,* or producing rots, *Botrytis cinerea,* produces cellulase involved in the degradation of plant material from the compostation substrates, synthetize 6-pentyl-pyrone, a volatile compound which is, in the same time, antifungal and odorant, and accelerates the formation of suppressive compost from fir tree bark.

2. Strain of *Trichoderma harzianum* Td50b NCAIM (P) F 001412, according to claim 1, **characterized in that** it has been selected from natural isolates using a high throughput screening process consisting of the following steps: aseptic distribution of an agarized medium, which contains as main carbon source a derivative of cellulose, in a plate with 24 wells, inoculation of agarized medium with 5 tested isolates, random distributed, and maintaining un-inoculated wells as controls; coverage with a sterile plastic film which allows the exchange of gases, incubation for 3 days at 25°C; deposition on the 24 well plates, on which were developed the tested isolates, over another plate with 24 wells with an agarized media, on which was grown for 2 days a microscopic fungus, phytopathogen agent, toward is tested the antagonism of the isolates; incubation for 5 days at 25°C of the reunited plates, with the plates with isolates of *Trichoderma* over the plates with phytopathogenic fungus for testing, separated by a plastic film which allow the gas exchange; determination of the phytopathogen agent growth by image analysis of the well plates, taken by a photodocumentation system, and calculation of the inhibition percent with the formula: $(A_1- A_n)/A_1$ x 100, where $A_1$ is the surface covered by the phytopathogen agent on the untreated control, and An is the surface covered by the phytopathogen agent in one of the experimental treatments wherein was confronted with the volatiles compounds produced by one of the tested isolates; statistical analysis of the variance and identification of the eventual isolates active due to the production of volatiles compounds; flooding of the agarized media on which were grown the isolates of *Trichoderma* with a solution of Red Congo 0.1 % and, after 5 min, removal of the solution of Red Congo and washing with a solution of 1 M NaCl for 20 min; revealing of the clear zones of cellulolysis around the colonies of *Trichoderma* producing cellulases.

3. Controlled-release composition based on *T. harzianum* Td50b **characterized in that** consists of 70 parts of hydrolyzed sawdust, 10 parts of flour, 4.8 parts of ethyl esters of fatty acids, 4 parts of polyvinyl alcohol, and 3.5 parts lecithin, 0.75 parts of potassium soap, 0.45 parts of glycerol, 0.4 parts triglycerides, residual water till 100 parts and min. $10^6$ cfu Td50b.

Europäisches
Patentamt

European
Patent Office

Office européen
des brevets

## EUROPEAN SEARCH REPORT

**Application Number**

EP 12 46 4023

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | REY M ET AL: "Improved antifungal activity of a mutant of Trichoderma harzianum CECT 2413 which produces more extracellular proteins", APPLIED MICROBIOLOGY AND BIOTECHNOLOGY, vol. 55, no. 5, May 2001 (2001-05), pages 604-608, XP002720911, ISSN: 0175-7598 * the whole document * | 1-3 | INV. C12N1/14 A01N63/00 ADD. C12R1/885 |
| X,D | WO 95/20879 A2 (HORTICULTURE AND FOOD RESEARCH [NZ]; US AGRICULTURE [US]; HILL ROBERT) 10 August 1995 (1995-08-10) * the whole document * | 1-3 | |
| T | ANONYMOUS: "Activator fungic pe baza de Trichoderma pentru producere de composturi supresive si odorante destinate culturilor ornamentale", SC Corax Bioneer SA , 3 December 2013 (2013-12-03), XP002720912, Retrieved from the Internet: URL:http://coraxbionerceu.ro/multi/wp-content/uploads/2013/12/Prezentare-TRICH-ODOR.pdf [retrieved on 2014-02-26] * the whole document * | 1-3 | |

TECHNICAL FIELDS SEARCHED (IPC)

C12N
C12R
A01N

The present search report has been drawn up for all claims

| Place of search | Date of completion of the search | Examiner |
|---|---|---|
| The Hague | 26 February 2014 | Devijver, Kristof |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 12 46 4023

This annex lists the patent family members relating to the patent documents cited in the above-mentioned European search report.
The members are as contained in the European Patent Office EDP file on
The European Patent Office is in no way liable for these particulars which are merely given for the purpose of information.

26-02-2014

| Patent document cited in search report | | Publication date | Patent family member(s) | | Publication date |
|---|---|---|---|---|---|
| WO 9520879 | A2 | 10-08-1995 | AU | 1547495 A | 21-08-1995 |
| | | | EP | 0743821 A1 | 27-11-1996 |
| | | | JP | H09508899 A | 09-09-1997 |
| | | | NZ | 250838 A | 27-05-1998 |
| | | | WO | 9520879 A2 | 10-08-1995 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1400586 B1
- US 7070984 B2
- US 4900348 A
- TW I287535 B
- RO 127471 A1
- WO 9520879 A2

**Non-patent literature cited in the description**

- **SAHAY-BAGNON et al.** *Process Biochem.,* 2000, vol. 36, 103-109
- **WILLIAM et al.** *Appl. Environ. Microbiol.,* 2003, vol. 69, 4190-4191
- **REITHNER et al.** *Fungal Genet. Biol.,* 2005, vol. 42, 749-760
- **COSKUNTUNA ; ÖZER.** *Crop Protection,* 2008, vol. 27, 330-336
- **BELL et al.** *Phytopathology,* 1982, vol. 72, 379-382
- **GHOSE.** *Pure Appl. Chem,* 1987, vol. 59, 257-268
- **KOVACS et al.** *Enz. Microbiol., Technol.,* 2008, vol. 43, 48-55
- **SERRANO-CARREÓN et al.** *Biotechnol. Lett.,* 2004, vol. 26, 1403-1406
- **KALYANI et al.** *Appl. Microbiol. Biotechnol.,* 2000, vol. 53, 610-612
- **SIMON et al.** *Soil Biol Biochem.,* 1988, vol. 20, 263-264
- **VAN DER PLAATS-NITERINK.** Monograph of the genus Pythium. Institute of the Royal Netherlands Academy of Sciences and Letters, 1981, 164-167